# EUROPEAN PATENT SPECIFICATION

(11) **EP 1 755 954 B1**
(45) Date of publication and mention of the grant of the patent: **28.04.2010**
(21) Application number: 05744250.1
(22) Date of filing: 24.05.2005
(51) Int. Cl.: B65B 9/02, B65B 51/28

(54) **FORM-FILL-SEAL PROCESS**
FORM-FILL-SEAL-VERFAHREN
PROCEDE DE FORMAGE-REMPLISSAGE-FERMETURE

(30) Priority: 24.05.2004 GB 0411543
(43) Date of publication of application: 28.02.2007
(73) Proprietor: Transdermal Technology & Systems (TTS) Limited, Windsor Berkshire SL4 5UB (GB)
(72) Inventor: Mark Rupert Tucker, Oxfordshire OX7 3NS (GB); Samuel Radcliffe Sneddon, Windsor, Berkssshire SL4 5UB (GB); Gareth Nigel Tempest, Richmond, North Yorkshire DL10 3BG (GB)
(74) Representative: Lord, Hilton David
(86) International application number: PCT/GB2005/002038
(87) International publication number: WO 2005/115843

(56) References cited:
- US-A- 3 405 502
- US-A- 4 782 647

## Description

The present invention relates to a process for the forming, filling and sealing of pouch products, such as reservoir transdermal patches, and to suitable apparatus for carrying out such processes.

Transdermal patches generally take one of two forms; matrix or reservoir. Matrix patches generally consist of an impermeable backing material having an adhesive on one side, and wherein the adhesive also contains the active substance for transdermal administration. Once the patch has been applied to the skin, the active substance leaches from the adhesive into the skin. However, such patches suffer from the dual problems of loading sufficient active substance into the adhesive, and subsequent high levels of retention of the active substance in the adhesive, rather than leaching out into the skin.

By contrast, reservoir patches have a reservoir of active substance formed between the impermeable backing material and the adhesive, which merely needs to allow passage of the substance. Problems with low loading and high retention are not generally encountered. Typically, these patches are illustrated by EP-A-336543.

Reservoir construction manufacturing processes do not affect the technical performance of the finished product in the same way that drug-in-adhesive manufacturing processes do. Several elements of the drug-in-adhesive manufacturing process can cause significant inter and intra-batch load variabilities in the active drug content, and can lead to significant variability in a wide range of *in* vitro performance characteristics. Causal factors include: curing of the adhesive at temperature; component dimensions; and, homogeneity of the active-drug matrix.

Volatile or toxic substances, such as nicotine and fentanyl, require minimal product loss during manufacture and shelf life, if they are to remain efficacious and safe under the recommended terms of use, and active loading contents at manufacture should substantially correspond with the loading contents when administered.

The processes for the production of reservoir patch, or pouch type, constructions yield significantly better intra and inter-batch results. These processes are also often better suited to volatile or toxic active compounds, as they encapsulate the active at the point of manufacture, and only release the majority of their load when administered to the patient. Within reservoir type manufacturing processes, it is recognised that continuous motion processes can provide significant benefits over intermittent processes, in that the continuous processes require fewer moving parts. Wear is greater on intermittent processes and cutter-life is shorter, so less maintenance and often lower component replacement costs arise with continuous processes. They also generate a higher component material yield than intermittent processes and are, therefore, more desirable to operate for volume production.

WO 99/52513 discloses a patch manufacturing process which is continuous and which generates a low component materials waste level. The process is a form-fill-seal process that may be in either the vertical or horizontal plane and incorporates flexibility of tooling, allowing a range of patch sizes to be manufactured. The process uses a plurality of opposing rollers in the vertical plane to create the vertical seals across a series of lanes, such that there is always one more vertical roller than there are lanes. Two opposing horizontal double edged jaws, whose length extends to cover the total width of the number of lanes, create the horizontal seal. The process enables the machine capacity to be expanded, by increasing the number of lanes, as required, with minimal additional tooling.

While this process allows versatility for the development of different sized products for pilot scale or early commercial scale production volumes, there are several key restrictions to this technology.
1. The patches that are created are square or rectangular and have angular corners. The cosmetic appearance cannot be changed automatically in-line or within this process, necessitating a subsequent die-cut function at a separate station, post assembly, which would require bandoliering the resultant web, as well as a print registration process to synchronise the web with the die-cut stations.
2. Primary packaging is a laborious process. For small volumes this can be done off-line with semi-automatic equipment, manually filling pre-formed pouches with the individual patches. This results in a significant work in process production bottleneck and large unit costs. For larger volumes, an automatic process can be used to pouch the patches, employing a separate machine post assembly with a 'pick & feed' or similar placement mechanism. This function tends to have a lower cycle speed, resulting in a production bottleneck, as well as generating additional costs, and there is a risk of damaging the patches when selecting them for packaging.
3. It is not possible to incorporate a tear-tab into the patch during primary production. Transdermal products include this characteristic in their specification. Examples include a separation mechanism achieved by placing the patch on a larger piece of material which can be removed from the patch relatively easily, or incorporating a 'crack & peel', 'butterfly' or similar effect, integrated within the release liner, so that the patch may be separated from the release liner prior to application. Such effects can contribute to increased product losses over time, so that it is generally preferable to ensure that the integrity of the release liner is maintained across the entire active surface area of the patch. This can be achieved by providing a kiss-cut across one or more corners of the patch individually, or on the web as a whole, at a subsequent station to the primary process. Again, this requires product registration to maintain synchronisation, and suffers from similar drawbacks to those associated with secondary pouching.
4. The process disclosed in WO 99/52513 is dependent on the contact surface grip between the horizontal jaws and vertical rollers to drive the materials through the process, as there is no independent web drive unit. In order to maintain constant pressure and prevent slippage, these surfaces are etched with a pattern, the specification of which is determined by the materials in use. This results in an obvious pattern within the seal which may not be cosmetically desirable. The level of pressure control that can be applied to the vertical rollers and horizontal jaws is limited, without either damaging the web materials or reducing drive friction. If these pressures are reduced too much, or rollers without any surface etching are used, the ability to maintain constant web friction is reduced, on the one hand, or damage to the appearance of the material can occur, on the other. Both are commercially undesirable and can affect technical performance of the product.
5. More significantly, by the very nature of the technique, the web can wander throughout the entire process. Whilst the effect can be minimised by ensuring the supply reels are of consistent quality and the sealing rollers are linearly aligned, inconsistencies in the reel specifications can create wander that has to be accommodated within the sealing and cutting process. This results in finished products which can vary in size, depending on the aforementioned variables. As a consequence, non-destructive sampling e.g. by weight, is not reliable, and all sampling is necessarily destructive.
6. While the process allows flexibility in selecting patch size, loading contents, machine throughputs and supply reel specifications, this flexibility can be detrimental in large scale manufacture, owing to the human error introduced thereby. In addition, both intra- and inter-batch variations can occur, according to machine or reel conditions at the time.
7. The degree of control that can be applied to, or measured at, the surface of the rollers or jaws is restricted, so that the provision of drive to the web materials can only be achieved if patterns are etched onto the sealing area.
8. Embossing or debossing of any delicate forms is extremely difficult and can only be achieved in cooperation with print registration or off-line.
9. Trimming of the patch or pouch edges to coincide with the edge of the seal is intrinsically difficult to synchronise. Excessive web wander can cause yield levels to reduce, and lead to inconsistent quality of the finished product.

The process disclosed in US-A-4,614,076 provides twin mono-blocks to create and seal the patches before cutting. The subsequent vertical cutting process separates the web into a plurality of vertical, parallel lanes prior to the transverse cutting station. Both cutting processes are separate from, and subsequent to, the sealing station. In order to synchronise location of finished product and to minimise inaccurate cutting, separate friction rolls are introduced between the sealing and cutting stations. As with WO 99/52513, the cutting process described is only capable of generating square or rectangular patches. The illustrated shaped patch can only be achieved by a separate die-cutting station, as discussed above. In addition, the degassing is such that a risk exists of the liquid escaping into the seal area.

Neither of the processes disclosed in US-A-4,614,076 and WO 99/52513 is capable of providing in-line sampling, without gaining access within the guarding' of the machinery, thereby leading to loss of significant volumes of finished product. All sampling is destructive. In-line reject management cannot be achieved without losing significant volumes of finished product. Primary packaging must be carried out off-line on a separate piece of machinery, and the de-gassing can lead to higher rejection rates.

Other processes are disclosed in US-A-4,845,926, US-A-4,004,399, US-A-3,210,908, FR-A-1,068,961 and US-A-4,769,974, but do not employ mono-block processes, so are liable to finished product specification variances. All except US-A-4,845,926 create large reservoir bellies, and neither US-A-3,210,908 nor US-A-4,769,974 are of a continuous process nature, resulting in dosing at an interphase, causing larger reservoir bellies.

WO 90/13487 discloses a process for manufacturing infusion packages, such as teabags. Piles of tea are dosed onto a horizontal web, which is drawn through a first sealing station and a second cutting station, before the bags are punched into receptacles. The tension in the web can lead to inaccuracies, and the process is not suitable for liquids.

A similar process, but where pairs of bags are interconnected by a tab, is disclosed in GB-A-2314312. Furthermore, US-A-4,782,647 discloses a process and an apparatus in accordance with the preambles of claims 1 and 25.

Surprisingly, we have now found that it is possible to provide the solution to many, if not all, of the above identified problems, by the use of a die-cutting monoblock.

Thus, in a first aspect, there is provided a one stage process for the preparation of sealed pouches, comprising:
Surprisingly, we have now found that it is possible to provide the solution to many, if not all, of the above identified problems, by the use of a die-cutting monoblock.

Thus, in a first aspect, there is provided a one stage process for the preparation of sealed pouches, comprising:
a first step of passing a plurality of webs between a first pair of cooperating, sealing rollers, at least one of the rollers having one or more forms thereon serving to define a pouch seal in cooperation with the second roller when the webs are passed therebetween;
a second step of passing the resulting sealed pouches between a second set of cooperating, cutting rollers, wherein one or more dies on at least one of the rollers serve to cut and sever the pouches from the skeleton of web material,
the second set of rollers being in register with the first set of rollers; and
a third step whereby severed pouches released from the second pair of rollers are captured and secured into outer pouches.

Pouches produced in accordance with the present invention are preferably produced in transdermal dosage form, oral dosage form, or implantable dosage form.

The processes of the art involve multiple cutting stations, serving to cut the web lengthways and crosswise. Accordingly, it is not possible to provide a shaped pouch other than by employing a further, die-cutting process. For example, the shaped pouch illustrated in US-A-4,614,076 cannot actually be produced by the cutting means illustrated in the patent. Although the forms on the first roller described therein may take any shape, the slitting rollers and the cutting station only permit the production of regular rectangles. In addition, the extra friction rollers and the existence of more than one cutting station introduces scope for error.

The process of the present invention is a one stage process, by which is meant that the various rollers of the invention are located in one station, with the sealed webs passing directly from one set of rollers to the next.

Thus, in a simple embodiment, the present invention provides a one stage process for the preparation of reservoir patches, and comprises feeding webs to a first pair of sealing rollers and, thence, directly to a second pair of die-cutting rollers, and directly into a packaging step, thereby avoiding most operator-related error, and substantially increasing overall sterility and reproducibility.

While it is possible for there to be provided supporting means, such as support rollers, between the two sets of rollers, it will generally be the case that no such support is required, as it is preferred to minimise the distance between the paired roller interfaces. The interfaces between the paired rollers may be at angles, such as to tension the web, although this is not generally preferred.

More generally, it is generally preferred to feed two webs to the first pair of rollers, with one web providing a backing material, generally impervious to the contents of the pouch, and the other web providing either a similar, or identical, backing material or, more preferably, a material having a desired set of properties.

The web having the additional properties may consist of one or more layers, and these layers may be fed, independently, into the apparatus of the invention, being sealed into laminate form on passing through the first set of rollers.

More preferably, the web is provided as a laminate so that, in the case of a reservoir patch, for example, this laminate may comprise an optional porous webbing, an adhesive and a release layer, which may be sealed, *via* the webbing, to the backing layer, on passing through the sealing rollers.

There is no need for calendaring, or otherwise embossing, the sheets of web material, as the sheets are sealed together at the first set of rollers, and pass without interruption into the second pair of rollers to be cut. Thus, there is generally no need to either grip or tension the skeleton containing the sealed pouches.

The sheets of web material are suitably provided on large rolls, and can be fed direct to the paired rollers. Laminated web material can be expensive and, as such, it may be preferred to split the sheet as it comes off the roll, in order to feed it direct to the forms on the first set of rollers, thereby to minimise waste between adjacent forms.

Where there are large numbers of forms, or the forms are small, for example, it may be preferable to provide strips of web material embracing two, three or four, but preferably two, forms, for ease of handling.

It will also be appreciated that it is not generally necessary to provide the backing material in such strip form, as the backing material is generally more economical to produce than the laminate and that, in addition, providing a single sheet of backing material also lends stability to the skeleton containing the pouches after passing through the first set of rollers.

In general, the webs may be supplied to the first pair of rollers in a conventional manner, such as is illustrated in WO99/52513.

Before sealing the pouches, any contents need to be positioned therein. In the event that the contents of the pouches are gaseous, then it may be sufficient simply to target a jet of the gas, or gaseous mixture, into the form for the pouch, in order to distend a belly, which is then sealed as the rollers continue to form the pouch. A similar technique may be employed for liquids or powders, whilst solids or gels, and other preparations capable of maintaining their form for at least a limited period, can be deposited on one or other web, or less preferably both, in register with the pouch forming function of the first pair of rollers. Such pouch contents may, in themselves, for example, be in capsule form, or the web upon which they are deposited may have a series of discretely formed adhesive patches, or overall adhesive layer, upon which the pouch contents may be deposited, in order to secure them to the web prior to sealing the incipient pouch.

It is a particular advantage of the present invention that it is possible to produce reservoir patches with minimal bellies. While pockets and other forms of recess are discussed herein, which are intended to accommodate such bellies, it will be appreciated that the bellies may range from the virtually negligible to the substantial, depending on the intended contents of the pouch. A highly active drug that may be contained at high concentrations in a gel may only require a pouch thickness in the order of a millimetre or less, while a pouch adapted to contain insect larvae may need to be an order of magnitude, or more, greater, for example.

Other suitable forms of pouch contents include drug solutions, such as aqueous gels of nicotine, suitably thickened, to permit deposition onto the web and minimal spreading prior to pouch formation.

On passing between the first pair of sealing rollers, the web materials are conjoined, with integral seals defining discrete pouches being formed on passage between the rollers.

In one embodiment, one of the rollers may simply be plain, whilst the opposing roller has one or more raised shapes about its periphery to encompass the volume of the pouch, and wherein the raised walls define the seal. These raised walls may extend as far as is desired towards an adjacent form, for example, in which case the area to receive the belly of the pouch may instead be considered to be a pocket in the roller.

In an alternative embodiment, pockets are provided in both rollers to accommodate the belly of the pouch.

It will also be appreciated that all or part of the area on one or both of the rollers defining the seal may be ridged or dimpled in order to assist in seal formation and/or gripping the web(s).

The seal itself may simply be formed by pressure, or may be effected by pressure sensitive or contact adhesive. More preferably, the seal is generated by the effect of heat, so that a combination of pressure and heat may serve to weld plastics components of the two webs together, for example, or to secure the two webs together *via* a heat activatable adhesive.

After passing through the first pair or rollers, the conjoined web may be considered to be a skeleton containing discrete, sealed pouches. The seals may be localised to the individual pouches, or may continue between pouches. In one embodiment, the seals are continuous between pouches in a lengthwise direction relative to the direction of travel.

The skeleton then passes between the second set of rollers. The second set of rollers is in registration with the first set of rollers, and preferably shares the same drive as the first set of rollers.

Registration may be effected by any suitable means known in the art, and is preferably effected mechanically, such as by a suitable gear train. In one embodiment, a contra-rotating roller is positioned in contact with a roller from each of the two pairs of rollers of the invention, and registration can be ensured by meshing matched teeth on the rollers. This can serve to ensure exact registration of sealing and subsequent cutting as, if the cutting rollers are not in exact registration with the sealing rollers, then meshing with the contra-rotating roller is impeded, and suitable restorative action can be taken.

As with the first pair of rollers, one of the second pair of rollers can be plain, with a cutting pattern on the other roller serving to cut through the web against the plain roller. Also as before, the second roller may have pockets to accommodate the pouch, so as not to force the skeleton away from the roller and to lead to any inaccuracy in cutting of the pouch. Such pockets may be generated by raising walls, with peripheries or flanges on the walls serving as cutting surfaces against which the template cutting edges of the opposing roller can act. A pocket within the cutting template is preferably provided, to accommodate the belly of the pouch.

It is not necessary, nor particularly desirable, that the pockets on either roller serve to engage the pouch. Instead, it is desirable that the pouch pass between the rollers without any significant interaction therewith, other than to be cut, and separate the pouch from the skeleton.

In a particularly preferred embodiment, the path between the two pairs of rollers is substantially vertical, such that the skeleton drops from the first pair of rollers into engagement with the second pair of rollers. By "substantially vertical" is meant that the process proceeds in the direction of gravity and that, if desired, the effects of gravity can be used assist the process. In this regard, an angle of 45° from the horizontal is the minimum angle, and is more preferably at least 80°, more preferably 85°, and is particularly preferably 90° ± 1°. Completely vertical is most preferred, in many circumstances, within tolerance.

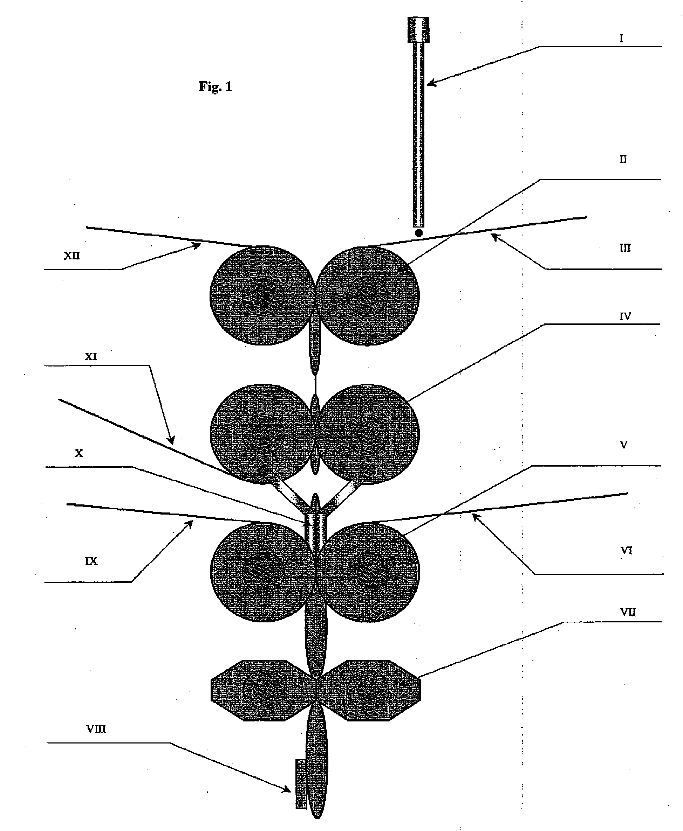

In such an embodiment, the contents of the pouch may be dropped, or dispensed, into the part-formed pouch, prior to completion of the seal, as illustrated in the accompanying Figure 1, for example. Suitable dropping or dispensing means may be employed. Where the contents are free flowing, flow control and/or metering means are preferably associated with the dropping or dispensing means. It will be appreciated that such means may also be associated with contents dispensing means in other aspects of the invention.

This embodiment can be especially efficient with flowing contents, such as powders and liquids, as the pouch remains open with the opening facing upwards, while the webs are fed in over the rollers, until the final sealing, and the dose can be metered such that the pouch fills as it is formed. In this way, there is little danger of spillage, while the pouch is capable of being filled, substantially completely, without there being an excess of air that is trapped or that needs to be expelled in some form.

It will also be appreciated that it is possible to employ other angles, including the complete opposite, whereby the skeleton passes upwards from the first pair of rollers, but this will generally entail guiding means, where the pathway is below the horizontal, or not much above, and may even necessitate friction rollers where the pathway is much above horizontal. It is particularly preferred to avoid having to use the former and more particularly preferred to avoid the necessity for the latter.

After passing through the second pair of rollers, the skeleton may be drawn away, for further use or disposal.

In a preferred embodiment, materials waste levels are no greater than 30%, after cutting.

In order to ensure separation of the pouches from the skeleton, either because the pouches were kiss-cut, or simply to ensure the efficacy of the cutting operation, punches may be employed to separate the pouches from the web skeleton.

The nature of such punches, where employed, is not critical to the present invention, but may take the form, for example, of a punch being temporarily extruded from one of the rollers against the direction that the web is being removed, thereby to create tension between the pouch and the skeleton, if there is any remaining contact between the pouch and skeleton.

Such punch means within the roller can be provided by the use of a suitable cam, for example.

The punch means, where provided, may be provided in either roller, and will generally correspond to at least 40 percent of the surface area of one side of the pouch formed by the cutter. Preferably, the punch face area corresponds to up to 90 percent, or more, of the pouch surface area.

It has been particularly surprisingly found that the whole process of pouch manufacture can be dramatically simplified by the incorporation of a primary packaging operation after the forming of the pouches, so that, as the pouches are formed, they are dispensed directly into a partially preformed container, or outer pouch, which can then be sealed or closed immediately upon receipt of the pouch. This is especially beneficial where it is desired to maintain sterile, or substantially sterile conditions, or where the pouch is fragile, and benefits from lack of further handling.

The present invention provides a process wherein the one stage process comprises a third step whereby severed pouches released from the second pair of rollers are captured and secured into outer, or primary, pouches. In this context, "one stage" indicates an uninterrupted, or continuous, process, in that the pouches are not, for example, removed and stored before packaging. In particular, it is envisaged that the pouches are dispensed into the outer pouches as they are separated from the skeleton, or web.

In a preferred embodiment, the outer pouches are prepared by drawing together suitable sheets of retaining material so as to entrain the severed pouches.

Where the pouches are dropped into partially preformed outer pouches, then this may be effected by suitable sealing rollers defining a continuous outer seal on either side of the pouch, and preferably leaving clearance between the pouch and the seal. The size of the clearance is not critical to the present invention, but is preferably at least 3 - 5mm.

After lengthwise sealing, transverse sealing jaws serve to create the third of the four seals of the outer pouch and, once at least one severed pouch is located within the outer pouch area, progression of the retaining material sheets through the apparatus will then result in a closed outer pouch when the sealing jaws seal the pouch and create the third of the four seals for the subsequent pouch.

It will be appreciated that the apparatus to form the outer pouch may be adapted in any suitable manner, and that, for example, self-adhesive pouch walls may be dispensed from release webs to surround the severed pouches.

In another embodiment, the severed pouches may be dispensed on to a sheet of material that is to form a part of the outer pouch, and wherein the sheet of material is angled away from the vertical such that the severed pouch remains in place. This embodiment is useful, for example, where punches are used to separate the pouches from the skeleton. The outer pouches may then be formed by the addition of a second wall, which may either be continuous or discontinuous, the outer pouches then being severed at a subsequent cutting station.

In preparing the outer pouches, the decoration for these pouches may be added before or after the severed pouches are incorporated, although it is generally preferred to provide the decoration, such as printing, embossing, or Braille, beforehand, in order to avoid unnecessary pressure on the incorporated pouch.

It is a particular advantage of the present invention that it is possible to locate all of the steps of the process within one cycle of each other and preferably within no more than three cycles of each other. In this respect, one cycle corresponds to the maximum circumference of either of the form and cutting rollers.

It is also possible to incorporate tear tabs into the process of the invention, this being readily incorporated into the cutting templates in the second pair of rollers. A nick at the periphery of the pouch provides a ready tear, whilst a nick within the seal area provides a childproof tear area where the seal area must first be bent over before tearing at the nick.

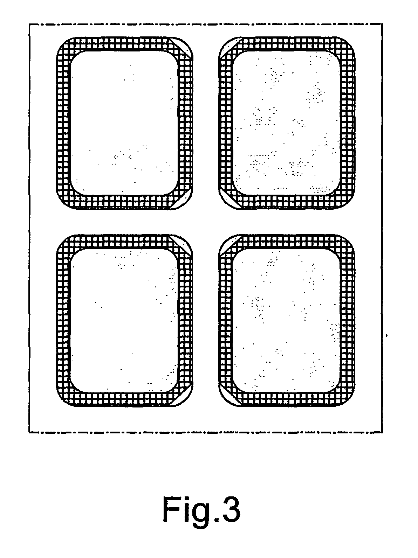

Also, as illustrated in Figure 3, for example, the seal area may not extend as far as the cutting area, so that it is possible to pull the two webs apart to reveal the contents of the pouch.

It is an advantage of the process of the present invention that there can be provided a continuous, fully integrated, manufacturing process for patches or pouches containing an active ingredient, supported within either a liquid or dry powder, that generates low levels of component material waste.

In a preferred embodiment, the pouches are created from two series of twin monoblocks of opposite natures, one male and one female, such that the first two opposing surfaces create a seal, and the second two opposing surfaces cut the shape of the finished product simultaneously. The sizes of such pouches may generally vary from a minimal size of, say, approximately 2.5cm² to a maximum size equivalent to the surface are of the mono-block circumferential surface area.

A high degree of pressure control is possible between the mono-blocks, thereby providing improved sealing and cutting quality, and the prevention of over-pressure or damage to component materials, as well as enabling an integrated drive for the web materials to be achieved with less moving parts and fewer separate stations than the art.

It is a particular advantage of the present invention that the process is capable of providing shaped, finished products, with fewer separate functions in the process.

It is a further advantage of the present invention that inter-batch and intra-batch variability is minimised, and consistency and repeatability of the finished product quality is enhanced.

In a preferred embodiment of the present invention, there is provided a fully integrated, in-line, primary packaging process. This can enable maintenance of atmospheric control in one unit for the whole process, and minimises non-sterile exposure or the necessity for handling. This may be further enhanced by the provision of a fully integrated facility for in-line primary packaging.

It is a further advantage that effective in-line reject and sampling management can be provided in a non-destructive fashion. For example, monitors may detect the edges of the webs and/or detect the extent of spread of filling substance, while success of cutting the pouches from the skeleton may be also be monitored, as can numerous other parameters readily apparent to those skilled in the art. An arm located in proximity to the cutting rollers and situated therebelow will generally suffice to clear a row of reject pouches, with the primary packaging apparatus readily halted while the row is rejected, thereby to prevent wastage of primary packaging.

In a preferred embodiment of the present invention, the shape of the finished pouch or patch within the web is defined at the same time as the sealing process is conducted, without any need for any subsequent offline or separate function to improve the aesthetics of the finished product. Rounded corners, or other such shapes, can be incorporated at this stage. The two series of mono-blocks are synchronised to ensure the cut occurs at the desired location, and eliminates the need for print registration. This integrated function reduces the cost of labour for the finished product by incorporating an in-line shaping process that has previously only been available, offline, as a separate function.

In this preferred embodiment, the integrated cutting mechanism within the second series of mono-block sealing tools limits the waste level of materials between individual pouches to that area outside of the seals of each pouch, thereby leading to a more consistent level of component material yield, irrespective of reel or machine conditions.

In a preferred embodiment of this invention, pre-calibrated standards are pre-set to measure and monitor effective loading of the patches or pouches, and to reject product if one of the conditions does not match. Since the primary packaging is supplied 'on-demand' in reaction to confirmed loading of the patches or pouches, a reject does not generate an empty primary pack, thus minimising wastage. Similarly, reel change splices or run-outs can be identified, so that any rejected product is isolated, without affecting machine running speeds, thereby minimising wastage levels and increasing finished product yield levels.

With the present invention, in-line sampling can be achieved without necessitating access to the 'guarded' area of the machine, and without interrupting flow. Due to consistent web placement and tighter tolerances, non-destructive sampling, such as check-weighing, can be employed.

Overprinting, in-line, is possible, without print registration, thereby allowing improved inventory management. Auto-insertion can be incorporated in-line at either the primary or secondary stage thus improving productivity.

In addition, by the nature of the integrated packing process, packs are readily rendered tamper-evident. In the preferred process of the invention, the primary packing is inherently tamper-evident, as the primary packing is sealed, and a broken seal is evidence of tampering. No tampering is possible during the process of the invention, without stopping the machinery, as the whole process is continuous and integral.

The continuous, integral and generally compact nature of the process of the invention enables the whole process to be carried out under a sterile, or clean, atmosphere, such as by flushing with nitrogen. Where the entire process is not hermetically sealed, it may be desirable to flush stages, such as filling, with clean, or sterile, gas. This process is referred to as degassing, in the art, and also covers evacuating the product. In the process of the invention, effective de-gassing may be achieved of both inner (patch or pouch) and outer primary (packaging) bags, in-line.

In preferred embodiments of this invention, overall component material utilisation, or yield level, exceeds 70% of the total size of the pouch.

Owing to the compactness and inherent lack of error of the process of the present invention, speeds in excess of the art (which typically run at up to 40 cycles/minute) are possible. Indeed, machine capacity is based on levels of 50 cycles/ minute or higher and may be increased by increasing cycle speed or according to the width of the mono-block cylinder and the number of lanes across the width, based on a standard size of patch or pouch reservoir.

Machine capacity may be adjusted in relation to the mono-block cylinders according to the size and number of patches or pouches produced per mono-block cycle. Each patch or pouch size is defined by the corresponding mono-block, such that each patch or pouch specification has its own dedicated mono-blocks for both sealing and cutting. Patch or sachet size variations are achieved by replacing both sets of mono-blocks as change parts for the same machine, which helps reduce product inconsistencies intra- and inter-batch. Each different set of mono-blocks may require unique supply reel web widths.

The 'bag-in-bag' process, or primary packaging, is preferably provided as an integrated function of the process, without any subsequent offline or separate function. This integrated function occurs in synchrony with the primary assembly process, thereby eliminating any work in progress, as a result of incorporating at least two separate processes into a single in-line function. Furthermore, product can be singled out and prepared for presentation to either primary or secondary packaging, and wherein the latter may also be introduced as an in-line function to eliminate any further work in progress.

In a preferred embodiment, a void is incorporated within the seal on both mono-blocks, so that part of the intended sealing area remains unsealed, thereby creating an integrated tear-tab within the seal. This allows the complete release liner to be peeled away from the bulk of the pouch or sachet to allow the user to separate the component materials with ease. This void does not affect the integrity of the seal, and does not cause any significant increase in product losses during storage.

It is advantageous that differential temperature controls are possible for each opposing mono-block, so as to be able to optimise sealing conditions for component materials.

Since both series of twin mono-blocks contribute to applying pressure that can be both controlled and monitored to provide consistent friction on the web, another advantage of the process of the present invention is that continuous motion can be maintained at consistent and constant pressure between both series of mono-blocks, which can be carefully controlled, monitored, and adjusted, to reflect the product specification requirements, without materially affecting the physical nature of the patch or pouch. It also allows a wider range of seal appearances to be applied to the seal surface without any loss of yield or machine throughput.

Given the ability to control the pressure applied at either, or all, of the primary or secondary mono-block or a subsequent station, delicate patterns may be embossed or de-bossed into the packaging material. Such patterns include logos or words as a labelling message for the consumer and may also include Braille.

It is advantageous that the number of operator variables are relatively few, such that the finished product specification is more consistent, and both the product and process can therefore be validated to higher pharmaceutical and cGMP regulatory standards.

It will be appreciated that the present invention is applicable to any product dispensable as a pouch, such as patches or pouches that essentially resemble miniature transdermal patches, for example, but that do not include an adhesive layer, a release liner or impermeable backing material. One such formulation may be sealed between two outer layers of membrane selected to allow controlled release of the formulation, and the resulting product used as an oral dosage form or implant, for example.

In another embodiment, patches or pouches comprise only backing material and removable release liner to reveal a skeleton web, through which the contents of the pouch, such as insect larvae, may pass freely. The adhesive layer is suitably limited to that area around the perimeter of the pouch or across the skeleton web. Alternatively, there may be no adhesive, and the pouch is adapted to be retained *in situ* by a breathable bandage, for example. The outer layers of material may, thus, serve merely to restrain the contents, rather than prevent intrusion.. The resulting product may be used as a mechanism to sort and transport live contents, such as larvae, and to provide containment when applied to a distal surface, such as a wound.

Self-diagnostics may be incorporated in to the apparatus of the invention in order to minimise machinery downtime and to assist in identifying any faults.

It will be appreciated that the present invention further provides apparatus suitable for use with a process as defined above, comprising a first pair of cooperating, sealing rollers, and means to feed a plurality of webs thereto, at least one of the rollers having one or more forms thereon serving to define a pouch seal in cooperation with the second roller when webs are passed therebetween, and a second set of cooperating, cutting rollers, wherein one or more dies on at least one of the rollers are suitable to cut and sever pouches generated by the first pair of rollers from the web material, wherein the second set of rollers is in register with the first set of rollers and severed pouches released from the second pair of rollers are captured and secured in outer pouches, and further comprising outer pouch forming means arranged to entrain severed pouches generated by the second pair of rollers.

It is preferred that one roller of the first pair has one or more raised forms thereon, comprising walls surrounding a pocket, suitable to encompass the volume of the pouch, and wherein the raised walls define the seal. In general, it is preferred to provide pockets in the rollers to accommodate the belly of the pouch.

As noted above, it is preferred that the second set of rollers shares the same drive as the first set of rollers.

Preferred apparatus has means to draw away the web skeleton after the second pair of rollers. Such apparatus may also comprise punches to separate the pouches from the web skeleton.

Preferred apparatus also comprises differential temperature controls are provided for each opposing roller.

Also, as noted above, each process step is preferably no more than three cycles removed from the next, and preferably no more than one cycle removed from the next.

Particularly preferred apparatus has all parts, from and including pouch content extrusion/dispensing through to sealing of the pouch, arranged in a single, atmospherically controlled unit. This single unit preferably also comprises the secondary packaging unit.

The present invention will be further illustrated with reference to the accompanying drawings, in which:
Figure 1 is a diagrammatic side view of a preferred embodiment of the invention, showing a schematic of a twin mono-block arrangement to continuously create pouches, together with integrated 'bag-in-bag' primary packaging whereby I = dose tube, II = upper sealing rollers, III = wrapping material B, IV = cutter V = lower sealing rollers, VI = outer pouch material B, VII = sealing jaws, VIII = Braille printing station, IX = outer pouch material A, X = nitrogen pipe injection, XI = waste removal, XII = wrapping material A;

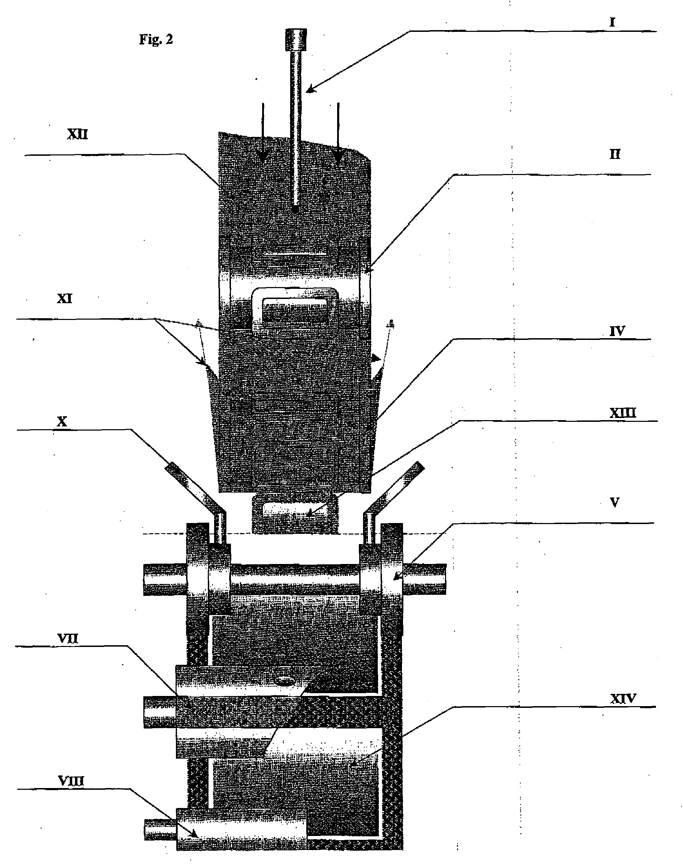

Figure 2 is a diagrammatic side view of the embodiment shown in Figure 1, but rotated through 90° whereby I = dose tube, II = upper sealing rollers, IV = cutter, XIII = inner pouch, V = lower sealing rollers, XIV = outer pouch, VIII = Braille printing, VII = sealing jaws, X = nitrogen pipe injection, XI = waste removal, XII = wrapping material A; and
Figure 3 shows a schematic of an arrangement of pouches, arranged as on a strip of web, and containing an integrated tear-tab void within the seal.

A particularly preferred embodiment of the present invention is shown in Figures 1 and 2, and provides two series of twin opposing mono-block cylinders to create a continuous manufacturing process for pouches and patches. The first series of mono-blocks seal together two parallel webs of material to create a reservoir that encapsulates the chosen formulation inserted between the two material webs. The second series of mono-blocks cut out the sealed patch or pouch template. In a third station, twin webs of outer pouch material are drawn into twin sealing rollers, collecting one or more pouches, subsequent sealing jaws serving to seal the outer pouch.

More specifically, pouch wrapping materials A and B are fed to the upper sealing rollers. In this embodiment, a dose tube is shown, dispensing a dose of pouch contents onto the wrapping material B. This is for illustrative purposes only, and it will be appreciated that the dose may be applied to either or both webs, and may be dispensed in any suitable fashion appropriate to the pouch contents. A nozzle to dispense liquids or powders will generally be located immediately above the sealing junction, contents being dispensed when a pouch is partially formed and sealed, so as to be able to receive the contents without spillage.

This and subsequent steps are performed under a sterile, or clean, atmosphere, such as a nitrogen blanket (not shown). While the atmosphere may be static, it is preferred to circulate and filter the atmosphere.

The wrapping materials are sealed in the areas defined by the forms on the sealing rollers as shown in Figure 2. It can be seen that the conjoined web materials with sealed pouches then drops to the next pair of rollers, where the pouches are cut out. In this arrangement, the web is guided by the raised walls at the perimeter of the cutter roller shown in Figure 2, but it is advantageous that no such guiding means is necessary, and the web, or skeleton, can drop straight into the cutter rollers, with a certain amount of traction lent by the cutting dies shown in Figure 2.

The pouches then fall into the third, bagging station, still under the nitrogen atmosphere. As shown in the Figures, the waste skeleton is removed at this stage, allowing the individual pouches to drop into the forming outer pouches.

The outer pouches are formed by feeding outer pouch material webs A and B into the lower sealing rollers. As shown in Figure 2, nitrogen can be blown into the outer pouch simultaneously to create a sterile atmosphere for the pouch, thereby removing the need for evacuation. A similar arrangement (not shown) is both possible and desirable when incorporating the pouch contents between the first pair of sealing rollers.

All rollers are shown providing sufficient clearance as not to compress the contents of the pouch. The sealing jaws seal the tops and bottoms of the outer pouches, the top of one being the bottom of the next, a further cutting station optionally being provided in-line within 1 - 3 cycles, and an optional boxing or other container-filling function being optionally located thereafter.

The Figures also demonstrate that the outer pouch material may be decorated. Here, the decoration is after pouches have been incorporated, although it is generally more preferred to decorate the outer materials prior to sealing.

## Claims

1. A one stage process for the preparation of sealed pouches, comprising:
a first step of passing a plurality of webs between a first pair of cooperating, sealing rollers (II), at least one of the rollers having one or more forms thereon serving to define a pouch seal in cooperation with the second roller when the webs are passed therebetween;
a second step of passing the resulting sealed pouches between a second set of cooperating, cutting rollers (IV), wherein one or more dies on at least one of the rollers serve to cut and sever the pouches from the skeleton of web material, the second set of rollers being in register with the first set of rollers; and
**characterised by** a third step whereby severed pouches released from the second pair of rollers are captured and secured into outer pouches (XIV).

2. A process according to claim 1, wherein one web is a laminate.

3. A process according to claim 1 or 2, wherein at least one web is split to reduce wastage between adjacent formes on the first set of rollers.

4. A process according to any preceding claims, wherein one web is basking material and has a width at least equivalent to the amount of forms on the first set of rollers that it is desired to use.

5. A process according to any preceding claim, wherein contents for the pouches are deposited on one or other web in register with the pouch forming function of the first pair of rollers (II).

6. A process according to any preceding claim, wherein the seal of each pouch is continuous between pouches in a lengthwise direction relative to the direction of feed.

7. A process according to any preceding claim, wherein the second set of rollers (IV) shares the same drive as the first set of rollers (II).

8. A process according to any preceding claim, wherein the path between the two pairs of rollers (II, IV) is substantially vertical.

9. A process according to any preceding claim, wherein, after passing through the second pair of rollers (IV), the skeleton is drawn away.

10. A process according to any preceding claim, wherein materials waste levels are no greater than 30%, after cutting.

11. A process according to any preceding claim, for the production of pouches suitable for transdermal, oral, or implantable administration.

12. A process according to claim 1, wherein the outer pouches (XIV) are prepared by drawing together and securing suitable sheets of retaining material and entraining the severed pouches therebetween.

13. A process according to claim 1 or 12, comprising adding decoration to the outer pouches (XIV) prior to incorporating the severed pouches.

14. A process according to claim 13, wherein the decoration is printing, embossing, or Braille.

15. A process according to any preceding claim, wherein each step of the process is no more than three cycles removed from the next.

16. A process according to claim 15, wherein each step is no more than one cycle removed from the next.

17. A process according to any preceding claim, wherein tear tabs are incorporated by suitable design of the first and/or second pair of rollers.

18. A process according to any preceding claim, wherein child-proofing is incorporated by suitable design of the first and/or second pair of rollers.

19. A process according to any preceding claim, which is conducted in a single, atmospherically controlled unit.

20. A process according to any preceding claim, wherein in-line, non-destructive reject and sampling management is provided.

21. A process according to claim 20, wherein single rows of pouches may be rejected.

22. A process according to claim 21, wherein the outer packaging step is interruptible on rejection of a row of pouches.

23. A process according to any preceding claim, wherein machine capacity is 50 cycles/ minute or higher.

24. A process according to any preceding claim, for the production of patches or pouches comprising backing material and removable release liner to reveal a skeleton web, through which the contents of the pouch may pass, an adhesive layer limited to that area around the perimeter of the pouch or across the skeleton web.

25. Apparatus suitable for use in accordance with any preceding claim, comprising:
a first pair of cooperating, sealing rollers (II), and means to feed a plurality of webs thereto, at least one of the rollers having one or more forms thereon serving to define a pouch seal in cooperation with the second roller when webs are passed therebetween;
a second set of cooperating, cutting rollers (IV), wherein one or more dies on at least one of the rollers are suitable to cut and sever pouches generated by the first pair of rollers from the web material, the second set of rollers (IV) being in register with the first set of rollers (II); and **characterised by** comprising
outer pouch forming means arranged to entrain severed pouches generated by the second pair of rollers.

26. Apparatus according to claim 25, wherein one roller of the first pair (II) has one or more raised forms thereon, comprising walls surrounding a pocket, suitable to encompass the volume of the pouch, and wherein the raised walls define the seal.

27. Apparatus according to claim 25 or 26, wherein pockets are provided in the rollers to accommodate the belly of the pouch.

28. Apparatus according to any of claims 25 to 27, wherein the second set of rollers (IV) shares the same drive as the first set of rollers (II).

29. Apparatus according to any of claims 25 to 28, comprising means to draw away web skeleton after the second pair of rollers (IV).

30. Apparatus according to any of claims 25 to 29, wherein punches are adapted to separate the pouches from the web skeleton.

31. Apparatus according to any of claims 25 to 30, arranged such that each process step is no more than three cycles removed from the next.

32. Apparatus according to claim 31, arranged such that each process step is no more than one cycle removed from the next.

33. Apparatus according to any of claims 25 to 32, wherein the rollers and any outer pouch forming means are arranged in a single, atmospherically controlled unit.

34. Apparatus according to any of claims 25 to 33, wherein differential temperature controls are provided for each opposing roller.

## Patentansprüche

1. Einstufiges Verfahren für die Herstellung von abgedichteten Beuteln, das die folgenden Schritte aufweist:
einen ersten Schritt des Führens einer Vielzahl von Bahnen zwischen einem ersten Paar von zusammenwirkenden Abdichtungswalzen (II), wobei mindestens eine der Walzen eine oder mehrere Formen darauf aufweist, die dazu dienen, eine Beutelabdichtung im Zusammenwirken mit der zweiten Walze zu definieren, wenn die Bahnen dazwischen geführt werden;
einen zweiten Schritt des Führens der resultierenden abgedichteten Beutel zwischen einer zweiten Reihe von zusammenwirkenden Schneidwalzen (IV), wobei ein oder mehr Werkzeuge auf mindestens einer der Walzen dazu dienen, die Beutel aus dem Skelett des Bahnmaterials zu schneiden und herauszutrennen, wobei die zweite Reihe von Walzen mit der ersten Reihe von Walzen ausgerichtet ist; und
**gekennzeichnet durch** einen dritten Schritt, mittels dem die herausgetrennten Beutel, die vom zweiten Paar von Walzen freigegeben werden, erfasst und zu äußeren Beuteln (XIV) fest verbunden werden.

2. Verfahren nach Anspruch 1, bei dem eine Bahn ein Laminat ist.

3. Verfahren nach Anspruch 1 oder 2, bei dem mindestens eine Bahn geteilt ist, um den Verlust zwischen benachbarten Formen auf der ersten Reihe von Walzen zu vorringern.

4. Verfahren nach irgendeinem der vorhergehenden Ansprüche, bei dem eine Bahn ein Grundmaterial ist und eine Breite aufweist, die mindestens der Größe der Formen auf der ersten Reihe von Walzen äquivalent ist, die man verwenden möchte.

5. Verfahren nach irgendeinem der vorhergehenden Ansprüche, bei dem der Inhalt für die Beutel auf der einen oder anderen Bahn in Übereinstimmung mit der Beutelherstellungsfunktion des ersten Paares von Walzen (II) abgelegt wird.

6. Verfahren nach irgendeinem der vorhergehenden Ansprüche, bei dem die Abdichtung eines jeden Beutels zwischen den Beuteln in einer Längsrichtung relativ zur Zuführrichtung kontinuierlich ist.

7. Verfahren nach irgendeinem der vorhergehenden Ansprüche, bei dem die zweite Reihe von Walzen (IV) den gleichen Antrieb gemeinsam mit der ersten Reihe von Walzen (II) nutzt.

8. Verfahren nach irgendeinem der vorhergehenden Ansprüche, bei dem der Verlauf zwischen den zwei Paaren von Walzen (II, IV) im Wesentlichen vertikal ist.

9. Verfahren nach irgendeinem der vorhergehenden Ansprüche, bei dem, nachdem es durch das zweite Paar von Walzen (IV) hindurchgegangen ist, das Skelett weggezogen wird.

10. Verfahren nach irgendeinem der vorhergehenden Ansprüche, bei dem das Materialabfallniveau nach dem Schneiden nicht größer als 30 % ist.

11. Verfahren nach irgendeinem der vorhergehenden Ansprüche für die Herstellung von Beuteln, die für eine transdermale, orale oder implantierbare Verabreichung geeignet sind.

12. Verfahren nach Anspruch 1, bei dem die äußeren Beutel (XIV) durch Zusammenziehen und festes Verbinden von geeigneten Schichten von Haltematerial und das Mitführen der herausgetrennten Beutel dazwischen hergestellt werden.

13. Verfahren nach Anspruch 1 oder 12, das den Schritt des Hinzufügens einer Verzierung auf den äußeren Beuteln (XIV) vor dem Einverleiben der herausgetrennten Beutel aufweist.

14. Verfahren nach Anspruch 13, bei dem die Verzierung ein Druck, eine Prägung oder eine Braille-Schrift ist.

15. Verfahren nach irgendeinem der vorhergehenden Ansprüche, bei dem ein jeder Schritt des Verfahrens nicht mehr als drei Zyklen vom nächsten entfernt ist.

16. Verfahren nach Anspruch 15, bei dem ein jeder Schritt nicht mehr als ein Zyklus vom nächsten entfernt ist.

17. Verfahren nach irgendeinem der vorhergehenden Ansprüche, bei dem Reißlaschen durch eine geeignete Konstruktion des ersten und/oder zweiten Paares von Walzen einverleibt werden.

18. Verfahren nach irgendeinem der vorhergehenden Ansprüche, bei dem eine Kindersicherung durch eine geeignete Konstruktion des ersten und/oder zweiten Paares von Walzen einverleibt wird.

19. Verfahren nach irgendeinem der vorhergehenden Ansprüche, das in einer einzelnen atmosphärisch gesteuerten Anlage durchgeführt wird.

20. Verfahren nach irgendeinem der vorhergehenden Ansprüche, bei dem ein zerstörungsfreies In-Line-Zurückweisungs- und Probenahmemanagement bereitgestellt wird.

21. Verfahren nach Anspruch 20, bei dem einzelne Reihen von Beuteln zurückgewiesen werden können.

22. Verfahren nach Anspruch 21, bei dem der Schritt der äußeren Verpackung bei Zurückweisung einer Reihe von Beuteln unterbrochen werden kann.

23. Verfahren nach irgendeinem der vorhergehenden Ansprüche, bei dem die Maschinenkapazität 50 Zyklen/Minute oder höher ist.

24. Verfahren nach irgendeinem der vorhergehenden Ansprüche für die Herstellung von Pflastern oder Beuteln, die ein Grundmaterial und ein entfernbares Trägermaterial aufweisen, um eine Skelettbahn zu offenbaren, durch die der Inhalt des Beutels gelangen kann, wobei eine Klebstoffschicht auf jenen Bereich um den Umfang des Beutels herum oder über die Skelettbahn hinweg begrenzt ist.

25. Vorrichtung, die für eine Verwendung nach irgendeinem der vorhergehenden Ansprüche geeignet ist, die aufweist:
ein erstes Paar von zusammenwirkenden Abdichtungswalzen (II) und ein Mittel für die Zuführung einer Vielzahl von Bahnen dorthin, wobei mindestens eine der Walzen eine oder mehrere Formen darauf aufweist, die dazu dienen, eine Beutelabdichtung im Zusammenwirken mit der zweiten Walze zu definieren, wenn die Bahnen dazwischen geführt werden;
eine zweite Reihe von zusammenwirkenden Schneidwalzen (IV), wobei ein oder mehr Werkzeuge auf mindestens einer der Walzen geeignet sind, um die durch das erste Paar von Walzen hergestellten Beutel aus dem Bahnmaterial zu schneiden und herauszutrennen, wobei die zweite Reihe von Walzen (IV) mit der ersten Reihe von Walzen (II) ausgerichtet ist; und **dadurch gekennzeichnet, dass** sie aufweist:
ein Formmittel für äußere Beutel, die angeordnet ist, um die durch das zweite Paar von Walzen hergestellten herausgetrennten Beutel mitzuführen.

26. Vorrichtung nach Anspruch 25, bei der eine Walze des ersten Paares (II) eine oder mehrere erhabene Formen darauf aufweist, die Wände aufweisen, die einen Hohlraum umgeben, geeignet für das Einschließen des Volumens des Beutels, und wobei die erhabenen Wände die Abdichtung definieren.

27. Vorrichtung nach Anspruch 25 oder 26, bei der Hohlräume in den Walzen bereitgestellt werden, um den Bauch des Beutels aufzunehmen.

28. Vorrichtung nach irgendeinem der Ansprüche 25 bis 27, bei der die zweite Reihe von Walzen (IV) den gleichen Antrieb gemeinsam mit der ersten Reihe von Walzen (II) nutzt.

29. Vorrichtung nach irgendeinem der Ansprüche 25 bis 28, die ein Mittel aufweist, um das Bahnskelett nach dem zweiten Paar von Walzen (IV) wegzuziehen.

30. Vorrichtung nach irgendeinem der Ansprüche 25 bis 29, bei der Stanzen angepasst sind, um die Beutel aus dem Bahnskelett zu trennen.

31. Vorrichtung nach irgendeinem der Ansprüche 25 bis 30, die so angeordnet ist, dass ein jeder Verfahrensschritt nicht mehr als drei Zyklen vom nächsten entfernt ist.

32. Vorrichtung nach Anspruch 31, die so angeordnet ist, dass ein jeder Verfahrensschritt nicht mehr als ein Zyklus vom nächsten entfernt ist.

33. Vorrichtung nach irgendeinem der Ansprüche 25 bis 32, bei der die Walzen und irgendein Formmittel für äußere Beutel in einer einzelnen atmosphärisch gesteuerten Anlage angeordnet sind.

34. Vorrichtung nach irgendeinem der Ansprüche 25 bis 33, bei der Differenztemperaturregler für jede Gegenwalze vorhanden sind.

## Revendications

1. Procédé continu pour la préparation de sachets scellés, comprenant :
une première étape, consistant à faire passer plusieurs bandes entre une première paire de rouleaux de scellement (11), au moins un des rouleaux comportant une ou plusieurs formes qui y sont appliquées, servant à définir un joint du sachet en coopération avec le deuxième rouleau lors du passage des bandes entre eux ;
une deuxième étape, consistant à faire passer les sachets scellés résultants entre un deuxième groupe de rouleaux de coupe à coopération (IV), une ou plusieurs filières sur au moins un des rouleaux servant à couper et à séparer les sachets du squelette du matériau en bande, le deuxième groupe de rouleaux étant aligné avec le premier groupe de rouleaux ; et
**caractérisé par** une troisième étape, lors de laquelle les sachets séparés libérés de la deuxième paire de rouleaux sont capturés dans des sachets externes (XIV) et fixés sur ceux-ci.

2. Procédé selon la revendication 1, dans lequel une bande est constituée par un stratifié.

3. Procédé selon les revendications 1 ou 2, dans lequel au moins une bande est divisée pour réduire le gaspillage entre des formes adjacentes sur le premier groupe de rouleaux.

4. Procédé selon l'une quelconque des revendications précédentes, dans lequel une bande constitue un matériau de support et a une largeur au moins équivalente au nombre de formes sur le premier groupe de rouleaux que l'on désire utiliser.

5. Procédé selon l'une quelconque des revendications précédentes, dans lequel le contenu pour les sachets est déposé sur une bande ou une autre bande, en coïncidence avec la fonction de formation des sachets de la première paire de rouleaux (II).

6. Procédé selon l'une quelconque des revendications précédentes, dans lequel le joint de chaque sachet est continu entre les sachets dans une direction longitudinale par rapport à la direction d'alimentation.

7. Procédé selon l'une quelconque des revendications précédentes, dans lequel le deuxième groupe de rouleaux (IV) a le même entraînement que le premier groupe de rouleaux (II).

8. Procédé selon l'une quelconque des revendications précédentes, sans lequel le trajet entre les deux paires de rouleaux (II, IV) est pratiquement vertical.

9. Procédé selon l'une quelconque des revendications précédentes, dans lequel, après le passage à travers la deuxième paire de rouleaux (IV), le squelette est retiré.

10. Procédé selon l'une quelconque des revendications précédentes, dans lequel les niveaux de gaspillage des matériaux ne sont pas supérieurs à 30% après la coupe.

11. Procédé selon l'une quelconque des revendications précédentes, pour la production de sachets appropriés pour une administration transdermique, orale ou par implantation.

12. Procédé selon la revendication 1, dans lequel les sachets externes (XIV) sont préparés en rapprochant et en fixant des feuilles appropriées de matériau de retenue et en entraînant les sachets séparés entre elles.

13. Procédé selon les revendications 1 ou 12, comprenant l'étape d'addition d'une décoration sur les sachets externes (XIV) avant l'incorporation des sachets séparés.

14. Procédé selon la revendication 13, dans lequel la décoration est appliquée par impression, gaufrage ou impression Braille.

15. Procédé selon l'une quelconque des revendications précédentes, dans lequel chaque étape du procédé est séparée de l'étape suivante de pas plus de trois cycles.

16. Procédé selon la revendication 15, dans lequel chaque étape est séparée de l'étape suivante de pas plus d'un cycle.

17. Procédé selon l'une quelconque des revendications précédentes, dans lequel des languettes d'arrachage sont incorporées par une conception appropriée des première et/ou deuxième paires de rouleaux.

18. Procédé selon l'une quelconque des revendications précédentes, dans lequel une sécurité pour les enfants est incorporée par une conception appropriée des première et/ou deuxième paires de rouleaux.

19. Procédé selon l'une quelconque des revendications précédentes, exécuté dans une seule unité à contrôle atmosphérique.

20. Procédé selon l'une quelconque des revendications précédentes, comprenant une gestion du rejet et d'échantillonnage en ligne, non destructive.

21. Procédé selon la revendication 20, dans lequel des rangées individuelles de sachets peuvent être rejetées.

22. Procédé selon la revendication 21, dans lequel l'étape d'emballage externe peut être interrompue lors de rejet d'une rangée de sachets.

23. Procédé selon l'une quelconque des revendications précédentes, dans lequel la capacité de la machine correspond à 50 cycles/minute ou plus.

24. Procédé selon l'une quelconque des revendications précédentes, pour la production de timbres ou de sachets comprenant un matériau de support et un revêtement détachable amovible pour révéler une bande de squelette, que peut traverser le contenu du sachet, et une couche adhésive limitée à la zone entourant le périmètre du sachet ou traversant la bande de squelette.

25. Appareil approprié à une utilisation selon l'une quelconque des revendications précédentes, comprenant :
une première paire de rouleaux de scellement à coopération (II) et un moyen pour y amener plusieurs bandes, au moins un des rouleaux comportant une ou plusieurs formes qui y sont appliquées, servant à définir un joint du sachet en coopération avec le deuxième rouleau lors du passage des bandes entre eux ;
un deuxième groupe de rouleaux de coupe à coopération (IV), une ou plusieurs filières sur au moins un des rouleaux étant appropriées pour couper et séparer des sachets produits par la première paire de rouleaux à partir du matériau en bande, le deuxième groupe de rouleaux (IV) étant aligné avec le premier groupe de rouleaux (II); et **caractérisé en ce qu'**il comprend
un moyen de formation de sachet externe destiné à entraîner les sachets séparés produits par la deuxième paire de rouleaux.

26. Appareil selon la revendication 25, dans lequel un rouleau de la première paire (II) comporte une ou plusieurs formes surélevées qui y sont appliquées, comprenant des parois entourant une poche, appropriée pour englober le volume du sachet, les parois surélevées définissant le joint.

27. Appareil selon les revendications 25 ou 26, dans lequel des poches sont agencées dans les rouleaux pour recevoir le bombement du sachet.

28. Appareil selon l'une quelconque des revendications 25 à 27, dans lequel le deuxième groupe de rouleaux (IV) a le même entraînement que le premier groupe de rouleaux (II).

29. Appareil selon l'une quelconque des revendications 25 à 28, comprenant un moyen pour retirer le squelette de la bande derrière la deuxième paire de rouleaux (IV).

30. Appareil selon l'une quelconque des revendications 25 à 29, dans lequel des poinçons sont adaptés pour séparer les sachets du squelette de la bande.

31. Appareil selon l'une quelconque des revendications 25 à 30, agencé de sorte que chaque étape du procédé est séparée de l'étape suivante de pas plus de trois cycles.

32. Appareil selon la revendication 31, agencé de sorte que chaque étape du procédé est séparée de l'étape suivante de pas plus d'un cycle.

33. Appareil selon l'une quelconque des revendications 25 à 32, dans lequel les rouleaux et un quelconque moyen externe de formation du sachet sont agencés dans une seule unité à contrôle atmosphérique.

34. Appareil selon l'une quelconque des revendications 25 à 33, dans lequel des contrôles de variation de la température sont fournis pour chaque rouleau opposé.
